# EUROPÄISCHE PATENTSCHRIFT

(11) **EP 4 519 248 B1**
(45) Veröffentlichungstag und Bekanntmachung des Hinweises auf die Patenterteilung: **10.12.2025**
(21) Anmeldenummer: 23700838.8
(22) Anmeldetag: 16.01.2023
(51) Int. Cl.: C07D 235/12, C07D 235/16, A61P 35/00, A61K 31/4184

(54) **VERFAHREN ZUR HERSTELLUNG VON 4-[5-[BIS(2-CHLORETHYL)AMINO]-1-METHYL-1H-BENZO[D]IMIDAZOL-2-YL]BUTANSÄUREALKYLESTERN UND FORMYLIERTEN DERIVATEN**
METHOD FOR THE PREPARATION OF 4- [5- [BIS (2-CHLOROETHYL) AMINO] -1-METHYL-1H-BENZO[D]IMIDAZOL-2-YL] BUTANOIC ACID ALKYL ESTERS AND FORMYLATED DERIVATIVES
PROCÉDÉ DE PRÉPARATION DE 4-[5-[BIS(2-CHLOROTHYL)AMINO]-1-MÉTHYL-1H-BENZO[D]IMIDAZOLE-2-YL] ESTERS D'ALKYL D'ACIDE BUTYRIQUE ET DÉRIVÉS FORMYLÉS

(30) Priorität: 04.05.2022 EP 22171496
(43) Veröffentlichungstag der Anmeldung: 12.03.2025
(73) Patentinhaber: Heraeus Precious Metals GmbH & Co. KG, 63450 Hanau (DE)
(72) Erfinder: KUNNARI, Tero, 63450 Hanau (DE); JOST, Steffen, 63450 Hanau (DE); RAUTER, Holger, 63450 Hanau (DE); GREIN, Jenin, 63450 Hanau (DE)
(74) Vertreter: Heraeus IP
(86) Internationale Anmeldenummer: PCT/EP2023/050792
(87) Internationale Veröffentlichungsnummer: WO 2023/213445

(56) Entgegenhaltungen:
- EP-A1- 2 617 716
- DE-A1- 102010 055 499
- US-A1- 2014 031 560

## Beschreibung

Die Erfindung betrifft ein Verfahren zur Herstellung von 4-[5-[Bis(2-chlorethyl)amino]-1-methyl-1*H*-benzo[d]imidazol-2-yl]butansäurealkylestern und formylierten Derivaten derselben.

Durch Esterhydrolyse können 4-[5-[Bis(2-chlorethyl)amino]-1-methyl-1*H*-benzo[d]imidazol-2-yl]butansäurealkylester zu 4-[5-[Bis(2-chlorethyl)amino]-1-methyl-1*H*-benzo[d]imidazol-2-yl]butansäure umgesetzt werden. 4-[5-[Bis(2-chlorethyl)amino]-1-methyl-1*H*-benzo[d]imidazol-2-yl]butansäure ist unter der Bezeichnung "Bendamustin" als antitumoraler Wirkstoff bekannt.

US 2014/0031560 A1 offenbart die Herstellung von 4-[5-[Bis(2-chlorethyl)amino]-1-methyl-1*H-*benzo[d]imidazol-2-yl]butansäurealkylester durch Umsetzung von 4-[5-[Bis(2-hydroxyethyl)amino]-1-methyl-1*H*-benzo[d]imidazol-2-yl]butansäurealkylester mit Thionylchlorid (SOCl₂) als Chlorierungsmittel. EP 2 617 716 A1 offenbart die Verwendung von Phosphorylchlorid (POCl₃) als Chlorierungsmittel zur Überführung der beiden Hydroxyethylgruppen von 4-[5-[Bis(2-chlorethyl)amino]-1-methyl-1*H*-benzo[d]imidazol-2-yl]butansäurealkylester in Chlorethylgruppen.

EP 2 468 716 A1 und ihre prioritätsbegründende DE102010055499 (A1) offenbaren ein Herstellungsverfahren für 4-[5-[Bis(2-chlorethyl)amino]-1-methyl-1*H*-benzo[d]imidazol-2-yl]butansäurealkylester. Als Edukte dienen dabei 4-[5-[Bis(2-hydroxyethyl)amino]-1-methyl-1*H-*benzo[d]imidazol-2-yl]butansäurealkylester, deren Hydroxyethylgruppen durch Reaktion mit Oxalylchlorid in Gegenwart von Dimethylformamid in Chlorethylgruppen überführt werden können unter Bildung von 4-[5-[Bis(2-chlorethyl)amino]-1-methyl-1*H*-benzo[d]imidazol-2-yl]butansäurealkylestern. Während stöchiometrisch, d.h. mit einem Molverhältnis von 2,0 mol Oxalylchlorid : 1 mol 4-[5-[Bis(2-hydroxyethyl)amino]-1-methyl-1*H*-benzo[d]imidazol-2-yl]butansäurealkylester gearbeitet werden kann, empfiehlt die EP 2 468 716 A1 bevorzugt mit einem stöchiometrischen Überschuss entsprechend einem Molverhältnis ≥2,6 mol, insbesondere von wenigstens 3,0 mol Oxalylchlorid pro mol 4-[5-[Bis(2-hydroxyethyl)amino]-1-methyl-1*H*-benzo[d]imidazol-2-yl]butansäurealkylester zu arbeiten. Die dabei genutzte in situ-Reaktion des Oxalylchlorids mit Dimethylformamid ist schwierig zu steuern mit Blick auf Vollständigkeit der Reaktion oder auf unerwünschte, störenden Einfluss nehmende Feuchtigkeit im Reaktionssystem. Arbeitet man gemäß der Lehre der EP 2 468 716 A1 mit besagtem als bevorzugt empfohlenen überstöchiometrischen Molverhältnis, erhält man zwar den betreffenden gewünschten 4-[5-[Bis(2-chlorethyl)amino]-1-methyl-1*H*-benzo[d]imidazol-2-yl]butansäurealkylester in guter Ausbeute, jedoch mit einer durch eine bislang unbekannte Verunreinigung verursachten, auch durch Behandlung mit Aktivkohle nur schwierig entfernbaren, unerwünschten Gelbfärbung. Die Intensität der Gelbfärbung steigt mit dem gewählten Molverhältnis von Oxalylchlorid zu 4-[5-[Bis(2-hydroxyethyl)amino]-1-methyl-1*H-*benzo[d]imidazol-2-yl]butansäurealkylester.

Aufgabe der Erfindung war es, ein verbessertes Herstellungsverfahren für 4-[5-[Bis(2-chlorethyl)amino]-1-methyl-1*H*-benzo[d]imidazol-2-yl]butansäurealkylester ausgehend von 4-[5-[Bis(2-hydroxyethyl)amino]-1-methyl-1*H*-benzo[d]imidazol-2-yl]butansäurealkylestern bereitzustellen.

Die Aufgabe kann gelöst werden durch ein Verfahren zur Herstellung eines 4-[5-[Bis(2-chlorethyl)amino]-1-methyl-1*H*-benzo[d]imidazol-2-yl]butansäurealkylesters durch Umsetzung eines 4-[5-[Bis(2-hydroxyethyl)amino]-1-methyl-1*H*-benzo[d]imidazol-2-yl]butansäurealkylesters mit Chlormethylendimethyliminiumchlorid der Formel ClCH=N(CH₃)₂Cl.

In einer ersten Ausführungsform kann das erfindungsgemäße Herstellungsverfahren mit einem praktisch stöchiometrischen Molverhältnis im Bereich von 2,0 bis 2,2 mol Chlormethylendimethyliminiumchlorid pro mol 4-[5-[Bis(2-hydroxyethyl)amino]-1-methyl-1*H-*benzo[d]imidazol-2-yl]butansäurealkylester mit guter Ausbeute an 4-[5-[Bis(2-chlorethyl)amino]-1-methyl-1*H*-benzo[d]imidazol-2-yl]butansäurealkylester und mit wenig Verunreinigung und wenig Nebenprodukt durchgeführt werden.

In einer zweiten Ausführungsform kann das erfindungsgemäße Herstellungsverfahren aber auch mit einem überstöchiometrischen Molverhältnis im Bereich von >2,2 bis beispielsweise 5 mol Chlormethylendimethyliminiumchlorid pro mol 4-[5-[Bis(2-hydroxyethyl)amino]-1-methyl-1*H-*benzo[d]imidazol-2-yl]butansäurealkylester durchgeführt werden. Mit Blick auf eine hohe Ausbeute an 4-[5-[Bis(2-chlorethyl)amino]-1-methyl-1*H*-benzo[d]imidazol-2-yl]butansäurealkylester ist die Wahl eines überstöchiometrischen Molverhältnisses allerdings weniger bevorzugt.

Bevorzugt handelt es sich bei dem im erfindungsgemäßen Verfahren mit Chlormethylendimethyliminiumchlorid umgesetzten 4-[5-[Bis(2-hydroxyethyl)amino]-1-methyl-1*H*-benzo[d]imidazol-2-yl]butansäurealkylester um einen C₁₋₄-Alkylester, insbesondere um 4-[5-*[Bis(2-hydroxyethyl)amino]-1-methyl-1H-benzo[d]imidazol-2-yl]butansäureethylester;* mit anderen Worten, bevorzugte Ausführungsformen des erfindungsgemäßen Verfahrens bestehen in dessen Durchführung mit Chlormethylendimethyliminiumchlorid und einem 4-[5-[Bis(2-hydroxyethyl)amino]-1-methyl-1*H*-benzo[d]imidazol-2-yl]butansäure-C₁₋₄-alkylester, insbesondere 4-[5-[Bis(2-hydroxyethyl)amino]-1-methyl-1*H*-benzo[d]imidazol-2-yl]butansäureethylester. Dementsprechend erhält man als Produkt einer bevorzugten Ausführungsform des erfindungsgemäßen Verfahrens einen 4-[5-[Bis(2-chlorethyl)amino]-1-methyl-1*H*-benzo[d]imidazol-2-yl]butansäure-C₁₋₄-alkylester, insbesondere den 4-[5-[Bis(2-chlorethyl)amino]-1-methyl-1*H*-benzo[d]imidazol-2-yl]butansäureethylester.

Chlormethylendimethyliminiumchlorid ist kommerziell erhältlich, beispielsweise bei Sigma-Aldrich. Es handelt sich um einen Feststoff.

Das erfindungsgemäße Syntheseverfahren kann zweckmäßig in einem aprotischen, wasserfreien (getrockneten) organischen Lösemittel mit einem E_{T}(30)-Wert im Bereich von 140 bis 193 kJ/mol respektive in einem Gemisch solcher Lösemittel durchgeführt werden. Beispiele für derartige Lösemittel umfassen chlorierte organische Lösemittel wie Dichlormethan und Chloroform, Ether wie Dioxan und Tetrahydrofuran, und Acetonitril.

Die beiden im erfindungsgemäßen Syntheseverfahren eingesetzten Edukte, das Chlormethylendimethyliminiumchlorid sowie ein 4-[5-[Bis(2-hydroxyethyl)amino]-1-methyl-1*H-*benzo[d]imidazol-2-yl]butansäurealkylester können in Lösung oder in Suspension miteinander reagieren, wobei im letzteren Falle eines der oder beide Edukte suspendiert vorliegen können. Die Zugabe der beiden Edukte kann in beliebiger Reihenfolge stattfinden, gelöst, suspendiert oder als Feststoff.

Beim erfindungsgemäßen Verfahren kann mit einem Anteil beispielsweise im Bereich von 30 bis 150 g Eduktmenge (Gesamtmenge beider Edukte) pro Liter organischen Lösemittel(gemisch)s gearbeitet werden.

Zweckmäßig ist es, dass eine Temperatur in der Reaktionsmischung beispielsweise im Bereich von 0 bis 60°C eingehalten wird, sowohl während der Zugabe der Edukte als auch während der Reaktion.

Die Reaktionsdauer kann beispielsweise im Bereich von 30 bis 360 Minuten liegen. Bevorzugt wird die Reaktionsmischung durchmischt, insbesondere gerührt.

Nach beendeter Reaktion kann Wasser zugegeben und ein pH-Wert beispielsweise im Bereich von 7 bis 10 durch Zugabe von Base eingestellt werden. Beispiele für verwendbare Basen umfassen Ammoniak, Alkalihydroxid, Alkalicarbonat und Alkalihydrogencarbonat.

Wird die Reaktion in einem nichtwassermischbaren Lösemittel durchgeführt, findet sich der im erfindungsgemäßen Verfahren gebildete 4-[5-[Bis(2-chlorethyl)amino]-1-methyl-1*H-*benzo[d]imidazol-2-yl]butansäurealkylester nach Wasserzugabe und pH-Wert-Einstellung in der organischen Phase. Anderenfalls kann der gebildete 4-[5-[Bis(2-chlorethyl)amino]-1-methyl-1*H-*benzo[d]imidazol-2-yl]butansäurealkylester nach Zugabe eines nichtwassermischbaren Lösemittels wie beispielsweise Dichlormethan in eine nichtwassermischbare organische Phase überführt und daraus mittels üblichen dem organischen Chemiker geläufigen Methoden gewonnen und gereinigt werden. Beispiele für solche Methoden umfassen Kristallisationsverfahren, Umfällungsverfahren mit einem Antisolvens und präparative chromatographische Verfahren.

Ein nach dem erfindungsgemäßen Verfahren hergestellter 4-[5-[Bis(2-chlorethyl)amino]-1-methyl-1*H*-benzo[d]imidazol-2-yl]butansäurealkylester kann einer Esterhydrolyse unter Bildung von 4-[5-[Bis(2-chlorethyl)amino]-1-methyl-1*H*-benzo[d]imidazol-2-yl]butansäure respektive deren Hydrochlorid-Salz unterzogen werden, beispielsweise analog zur aus EP 2 468 716 A1 bekannten Vorgehensweise.

Das erfindungsgemäße Herstellungsverfahren weist eine Reihe von Vorteilen auf im Vergleich zum aus EP 2 468 716 A1 bekannten Herstellungsverfahren:
1. Das Arbeiten mit Chlormethylendimethyliminiumchlorid als Chlorierungsreagens anstelle von Oxalylchlorid/Dimethylformamid ist aus Arbeitssicherheitssicht in mehrfacher Hinsicht vorzuziehen. Neben den Vorteilen einer Vermeidung des Umgangs mit den Gefahrstoffen Oxalylchlorid und Dimethylformamid kann eine mit der Reaktion von Oxalylchlorid mit Dimethylformamid einhergehende Exothermie und Gasbildung vermieden werden.
2. Das Arbeiten mit Chlormethylendimethyliminiumchlorid erlaubt eine akkurate Dosierung im Hinblick auf die Einstellung eines gewünschten stöchiometrischen oder überstöchiometrischen Molverhältnisses zwischen Chlormethylendimethyliminiumchlorid und 4-[5-[Bis(2-hydroxyethyl)amino]-1-methyl-1*H*-benzo[d]imidazol-2-yl]butansäurealkylester; ebenso ermöglicht es eine gegebenenfalls notwendig werdende Nachdosierung von Chlormethylendimethyliminiumchlorid.
3. Vermeidbarkeit respektive Reduzierbarkeit von Nebenreaktionen insbesondere bei Durchführung des erfindungsgemäßen Verfahrens bei einem praktisch stöchiometrischen Molverhältnis im Bereich von 2,0 bis 2,2 mol Chlormethylendimethyliminiumchlorid pro mol 4-[5-[Bis(2-hydroxyethyl)amino]-1-methyl-1*H*-benzo[d]imidazol-2-yl]butansäurealkylester.
4. Geringerer Verbrauch von Base bei der Einstellung des pH-Wertes nach Beendigung der Reaktion.

Wie schon vorerwähnt, kann das erfindungsgemäße Herstellungsverfahren in seiner zweiten Ausführungsform mit einem überstöchiometrischen Molverhältnis im Bereich von >2,2 bis beispielsweise 5 mol Chlormethylendimethyliminiumchlorid pro mol 4-[5-[Bis(2-hydroxyethyl)amino]-1-methyl-1*H*-benzo[d]imidazol-2-yl]butansäurealkylester durchgeführt werden. Bei Durchführung des erfindungsgemäßen Verfahrens in der zweiten Ausführungsform, beispielsweise mit einem Molverhältnis im Bereich von 2,6 bis 5 mol Chlormethylendimethyliminiumchlorid pro mol 4-[5-[Bis(2-hydroxyethyl)amino]-1-methyl-1H-benzo[d]imidazol-2-yl]butansäure-C₁₋₄-alkylester, speziell 4-[5-[Bis(2-hydroxyethyl)amino]-1-methyl-1H-benzo[d]imidazol-2-yl]butansäureethylester kann eine zur eingangs schon erwähnten Gelbfärbung analoge Gelbfärbung im Reaktionssystem beobachtet werden, welche mit steigendem stöchiometrischen Überschuss an Chlormethylendimethyliminiumchlorid zunimmt. Die die Gelbfärbung verursachenden Substanzen stellen Verfahrensnebenprodukte des erfindungsgemäßen Verfahrens in seiner zweiten Ausführungsform dar und konnten jetzt erstmalig als die bislang unbekannten und somit neuen Verbindungen aus der Gruppe der 4-[5-[Bis(2-chlorethyl)amino]-4-formyl-1-methyl-1*H*-benzo[d]imidazol-2-yl]butansäure-C₁₋₄-alkylester identifiziert respektive isoliert werden. Offenbar kann es bei Durchführung des erfindungsgemäßen Verfahrens in seiner zweiten Ausführungsform zu einer regioselektiven Formylierung des eigentlichen Verfahrenshauptproduktes in 4-Position seines Benzo[d]imidazol-Gerüsts kommen. Der entsprechende bislang unbekannte und somit neue 4-[5-[Bis(2-chlorethyl)amino]-4-formyl-1-methyl-1*H*-benzo[d]imidazol-2-yl]butansäureethylester konnte nach Abtrennung vom Verfahrenshauptprodukt, dem 4-[5-[Bis(2-chlorethyl)amino]-1-methyl-1*H*-benzo[d]imidazol-2-yl]-butansäureethylester, gewonnen und charakterisiert werden. Mithin handelt es sich bei 4-[5-[Bis(2-chlorethyl)amino]-4-formyl-1-methyl-1*H*-benzo[d]imidazol-2-yl]butansäureethylester um ein bei Durchführung des erfindungsgemäßen Verfahrens in seiner zweiten Ausführungsform gebildetes Verfahrensnebenprodukt. Aufgrund seiner strukturellen Verwandtschaft mit 4-[5-[Bis(2-chlorethyl)amino]-1-methyl-1*H*-benzo[d]imidazol-2-yl]butansäure kann eine antitumorale Wirkung auch für 4-[5-[Bis(2-chlorethyl)amino]-4-formyl-1-methyl-1*H*-benzo[d]imidazol-2-yl]butansäureethylester erwartet werden. Seine Aldehydgruppe als reaktive Funktionalität kann einen Ausgangspunkt für mannigfaltige intra- und intermolekulare Derivatisierungsreaktionen und damit zur Herstellung von antitumoral wirksamen Verbindungen bieten, beispielsweise auch zur Bildung von Antikörperkonjugaten.

Über das bei Durchführung des erfindungsgemäßen Verfahrens in seiner zweiten Ausführungsform gewählte Molverhältnis im Bereich von >2,2 mol bis beispielsweise 5 mol Chlormethylendimethyliminiumchlorid pro mol 4-[5-[Bis(2-hydroxyethyl)amino]-1-methyl-1*H-*benzo[d]imidazol-2-yl]butansäurealkylester, bevorzugt 4-[5-[Bis(2-hydroxyethyl)amino]-1-methyl-1*H*-benzo[d]imidazol-2-yl]butansäure-C₁₋₄-alkylester, speziell 4-[5-[Bis(2-hydroxyethyl)amino]-1-methyl-1*H*-benzo[d]imidazol-2-yl]butansäureethylester kann das Verhältnis zwischen dem Verfahrenshauptprodukt (4-[5-[Bis(2-chlorethyl)amino]-1-methyl-1*H-*benzo[d]imidazol-2-yl]butansäurealkylester, bevorzugt 4-[5-[Bis(2-chlorethyl)amino]-1-methyl-1*H*-benzo[d]imidazol-2-yl]butansäure-C₁₋₄-alkylester, speziell 4-[5-[Bis(2-chlorethyl)amino]-1-methyl-1*H*-benzo[d]imidazol-2-yl]butansäureethylester) und dem Verfahrensnebenprodukt (4-[5-[Bis(2-chlorethyl)amino]-4-formyl-1-methyl-1*H*-benzo[d]imidazol-2-yl]butansäurealkylester, bevorzugt 4-[5-[Bis(2-chlorethyl)amino]-4-formyl-1-methyl-1*H*-benzo[d]imidazol-2-yl]butansäure-C₁₋₄-alkylester, speziell 4-[5-[Bis(2-chlorethyl)amino]-4-formyl-1-methyl-1*H*-benzo[d]imidazol-2-yl]butansäureethylester) gesteuert werden. Während mit dem erfindungsgemäßen Verfahren gemäß seiner ersten Ausführungsform praktisch kein Nebenprodukt erhalten wird, kann bei einem im Bereich von >2,2 bis beispielsweise 5 liegenden Molverhältnis ein Gemisch aus Verfahrenshaupt- und Verfahrensnebenprodukt erhalten werden. Mit steigendem Molverhältnis steigt dabei der relative Anteil an Verfahrensnebenprodukt bis auf etwa 5 mol-% neben etwa 95 mol-% des Verfahrenshauptproduktes. Eine Steigerung des Molverhältnisses auf >5 mol Chlormethylendimethyliminiumchlorid pro mol 4-[5-[Bis(2-hydroxyethyl)amino]-1-methyl-1*H-*benzo[d]imidazol-2-yl]butansäurealkylester empfiehlt sich beim erfindungsgemäßen Verfahren gemäß seiner zweiten Ausführungsform nicht.

Ein als Verfahrensnebenprodukt des in seiner zweiten Ausführungsform durchgeführten erfindungsgemäßen Verfahrens erhältlicher 4-[5-[Bis(2-chlorethyl)amino]-4-formyl-1-methyl-1*H-*benzo[d]imidazol-2-yl]butansäurealkylester kann einem weiteren Verfahrensschritt in Form einer Esterhydrolyse unter Bildung von 4-[5-[Bis(2-chlorethyl)amino]-4-formyl-1-methyl-1*H-*benzo[d]imidazol-2-yl]butansäure unterzogen werden, beispielsweise in Anlehnung an die aus EP 2 468 716 A1 bekannte Vorgehensweise, worauf hiermit ausdrücklich Bezug genommen wird.

Es ist auch möglich, das erfindungsgemäße Verfahren gemäß seiner zweiten Ausführungsform durchzuführen und das Verfahrensnebenprodukt der Esterhydrolyse gemeinsam mit dem Verfahrenshauptprodukt zu unterwerfen. Mit anderen Worten, in dem Fall wird das erhaltene Produktgemisch aus besagtem Verfahrenshaupt- und Verfahrensnebenprodukt zunächst nicht aufgetrennt, sondern gemeinsam einer Esterhydrolyse unterworfen, ebenfalls beispielsweise in Anlehnung an die aus EP 2 468 716 A1 bekannte Vorgehensweise, worauf hiermit ausdrücklich Bezug genommen wird. Das dabei erhaltene Gemisch von 4-[5-[Bis(2-chlorethyl)amino]-1-methyl-1*H*-benzo[d]imidazol-2-yl]butansäure und 4-[5-[Bis(2-chlorethyl)amino]-4-formyl-1-methyl-1*H*-benzo[d]imidazol-2-yl]butansäure kann dann anschließend aufgetrennt werden mittels dem organischen Chemiker geläufiger Methoden wie beispielsweise Kristallisation und/oder präparativer chromatographischer Verfahren.

Die hierin mehrfach erwähnte Esterhydrolyse kann wie gesagt in Anlehnung an die aus EP 2 468 716 A1 bekannte Vorgehensweise durchgeführt werden. Dazu wird ein betreffender isolierter 4-[5-[Bis(2-chlorethyl)amino]-1-methyl-1*H*-benzo[d]imidazol-2-yl]butansäurealkylester, also ein entsprechender formylierter oder nichtformylierter Butansäurealkylester, oder ein nicht aufgetrenntes Gemisch beider vorzugsweise mit einer Säure versetzt. Als Säure dient dabei vorzugsweise eine anorganische Säure, insbesondere Salzsäure. Die Säure wird üblicherweise als konzentrierte Säure, zum Beispiel als konzentrierte Salzsäure, eingesetzt. Das Gemisch aus formyliertem und nichtformyliertem Ester kann mit der Säure versetzt werden, indem die Säure mit dem Ester(gemisch) oder mit einer Lösung dessen in einem geeigneten organischen Lösemittel vereinigt wird. Im Anschluss wird die erhaltene Mischung vorzugsweise bei einer Temperatur im Bereich von 10 bis 80°C, mehr bevorzugt bei einer Temperatur im Bereich von 15 bis 70°C und noch mehr bevorzugt bei einer Temperatur im Bereich von 20 bis 60°C gerührt. Die Reaktionsdauer beträgt vorzugsweise 30 Minuten bis sechs Stunden, mehr bevorzugt eine Stunde bis vier Stunden und noch mehr bevorzugt eine Stunde bis drei Stunden. Nach der Umsetzung können gegebenenfalls in der Mischung enthaltene organische Bestandteile, wie zum Beispiel Lösemittel oder Lösemittelreste, entfernt werden. Dies kann vorzugsweise durch Destillation dieser organischen Bestandteile geschehen. Die in der Mischung enthaltene Säure wird dann auf herkömmliche Weise von dem oder den hydrolysierten Estern entfernt, vorzugsweise destillativ abgetrennt. Als Rückstand verbleibt die betreffende substituierte Butansäure, also die formylierte substituierte Butansäure (4-[5-[Bis(2-chlorethyl)amino]-4-formyl-1-methyl-1*H*-benzo[d]imidazol-2-yl]butansäure) oder die nichtformylierte substituierte Butansäure (4-[5-[Bis(2-chlorethyl)amino]-1-methyl-1*H*-benzo[d]imidazol-2-yl]butansäure) oder das betreffende Gemisch aus formylierter substituierter Butansäure und nichtformylierter substituierter Butansäure.

4-[5-[Bis(2-chlorethyl)amino]-4-formyl-1-methyl-1*H*-benzo[d]imidazol-2-yl]butansäure zeigt eine antitumorale Wirkung, beispielsweise gegenüber solchen Krebsarten wie auch 4-[5-[Bis(2-chlorethyl)amino]-1-methyl-1*H*-benzo[d]imidazol-2-yl]butansäure. Im nachfolgend offenbarten Beispiel 3 konnte dies experimentell nachgewiesen werden. 4-[5-[Bis(2-chlorethyl)amino]-4-formyl-1-methyl-1*H*-benzo[d]imidazol-2-yl]butansäure und ihre pharmazeutisch verträglichen Salze, beispielsweise ein Chlorid, Phosphat, Sulfat, Acetat, Maleinat, Citrat oder Mesylat, zeigen schon im Hundertmikromol pro Liter niedrigen IC50-Konzentrationsbereich eine Krebszellen abtötende respektive eine das Wachstum von Krebszellen inhibierende Wirkung, beispielsweise bei Hautkrebs, Nierenkrebs, Lungenkrebs, Gehirntumoren und Bauchspeicheldrüsenkrebs. IC50 steht hier für "Inhibition concentration", bei der 50% der Krebszellen abgetötet werden.

Die Aldehydgruppe als auch die Carboxylgruppe von 4-[5-[Bis(2-chlorethyl)amino]-4-formyl-1-methyl-1*H*-benzo[d]imidazol-2-yl]butansäure können als reaktive Funktionalitäten Ausgangspunkte für mannigfaltige intra- und intermolekulare Derivatisierungsreaktionen und damit zur Herstellung von antitumoral wirksamen Verbindungen bieten, beispielsweise auch zur Bildung von Antikörperkonjugaten.

4-[5-[Bis(2-chlorethyl)amino]-4-formyl-1-methyl-1*H*-benzo[d]imidazol-2-yl]butansäure - genauer gesagt 4-[5-[Bis(2-chlorethyl)amino]-4-formyl-1-methyl-1*H*-benzo[d]imidazol-2-yl]butansäure in ihrer Funktion als aktive Wirksubstanz - und ihre pharmazeutisch verträglichen Salze können demgemäß wie im Folgenden aufgezählt Verwendung finden:
1. in der Medizin, insbesondere in der Humanmedizin, speziell Krebsmedizin;
2. bei der oder zur therapeutischen Behandlung von Krebs, insbesondere Hautkrebs, Nierenkrebs, Lungenkrebs, Gehirntumoren und Bauchspeicheldrüsenkrebs;
3. als Medikament gegebenenfalls in Kombination mit anderen Medikamenten, insbesondere als Medikament bei der oder zur therapeutischen Behandlung von Krebs, insbesondere Hautkrebs, Nierenkrebs, Lungenkrebs, Gehirntumoren und Bauchspeicheldrüsenkrebs;
4. als Arzneimittel, insbesondere als Arzneimittel bei der oder zur therapeutischen Behandlung von Krebs, insbesondere Hautkrebs, Nierenkrebs, Lungenkrebs, Gehirntumoren und Bauchspeicheldrüsenkrebs;
5. bei der oder zur Herstellung eines Arzneimittels, insbesondere eines Arzneimittels zur therapeutischen Behandlung von Krebs, insbesondere Hautkrebs, Nierenkrebs, Lungenkrebs, Gehirntumoren und Bauchspeicheldrüsenkrebs.

### Ausführungsbeispiel 1 (Umsetzung von Chlormethylendimethyliminiumchlorid mit 4-[5-[Bis(2-hydroxyethyl)aminol-1-methyl-1H-benzo[d]imidazol-2-yl]butansäureethylester im Molverhältnis 2,0 : 1):

3,5 g Chlormethylendimethyliminiumchlorid und 55,8 g Dichlormethan wurden in einem Kolben unter Rühren auf 5°C abgekühlt. Zu dieser Suspension wurde eine 1°C kalte Lösung von 5,0 g 4-[5-[Bis(2-hydroxyethyl)amino]-1-methyl-1*H*-benzo[d]imidazol-2-yl]butansäureethylester in 28,7 g Dichlormethan portionsweise zugegeben, danach auf 40°C erwärmt und für 9 h am Rückfluss gerührt. Danach wurde auf 10°C abgekühlt und 10 mL Wasser zugegeben, ein pH-Wert zwischen 8 und 10 mit 10 gew.-%iger wässriger Kaliumcarbonatlösung eingestellt, 10 min nachgerührt und das Zweiphasensystem in einen Scheidetrichter überführt. Die Dichlormethan-Phase wurde abgetrennt. Zur wässrigen Phase wurden 46 mL Dichlormethan zugegeben, ausgeschüttelt und die Dichlormethan-Phase abgetrennt. Die vereinigten Dichlormethan-Phasen wurden mit 15 mL konzentrierter Kochsalzlösung gewaschen und das Dichlormethan unter Vakuum und mittels Wärmezufuhr am Rotationsverdampfer entfernt. Der Rückstand wurde aus 10mL Ethylacetat umkristallisiert.

Man erhielt 4,92 g 4-[5-[Bis(2-chlorethyl)amino]-1-methyl-1*H*-benzo[d]imidazol-2-yl]butansäureethylester (= 89 % Ausbeute, bezogen auf die eingesetzten 5,0 g 4-[5-[Bis(2-hydroxyethyl)amino]-1-methyl-1*H*-benzo[d]imidazol-2-yl]butansäureethylester).

### Ausführungsbeispiel 2 (Umsetzung von Chlormethylendimethyliminiumchlorid mit 4-[5-[Bis(2-hydroxyethyl)amino]-1-methyl-1H-benzo[d]imidazol-2-yl]butansäureethylester im Molverhältnis 4,0 : 1, gefolgt von Esterhydrolyse und Produktgewinnung):

7,0 g Chlormethylendimethyliminiumchlorid und 55,8 g Dichlormethan wurden in einem Kolben unter Rühren auf 5°C abgekühlt. Zu dieser Suspension wurde eine 1°C kalte Lösung von 5,0 g 4-[5-[Bis(2-hydroxyethyl)amino]-1-methyl-1*H*-benzo[d]imidazol-2-yl]butansäureethylester in 28,7 g Dichlormethan portionsweise zugegeben, danach auf 40°C erwärmt und für 9 h am Rückfluss gerührt. Danach wurde auf 10°C abgekühlt und 10 mL Wasser zugegeben, ein pH-Wert zwischen 8 und 10 mit 10 gew.-%iger wässriger Kaliumcarbonatlösung eingestellt, 10 min nachgerührt und das Zweiphasensystem in einen Scheidetrichter überführt. Die Dichlormethan-Phase wurde abgetrennt. Zur wässrigen Phase wurden 46 mL Dichlormethan zugegeben, ausgeschüttelt und die Dichlormethan-Phase abgetrennt. Die vereinigten Dichlormethan-Phasen wurden mit 15 mL konzentrierter Kochsalzlösung gewaschen. Anschließend wurde die gewaschene Dichlormethan-Phase mit 5,78 g 32 gew.-%iger Salzsäure versetzt und das Dichlormethan unter Vakuum und mittels Wärmezufuhr am Rotationsverdampfer entfernt. Es wurden weitere 23,8 mL 32 gew.-%ige Salzsäure zugegeben und es wurde bei 40°C Innentemperatur 90 min gerührt. Nach Abkühlen auf 20°C wurden 0,58 g Aktivkohle zugegeben und es wurde 20 min gerührt, anschließend die Aktivkohle abfiltriert und mit 24,8 mL Wasser nachgespült. Danach wurde bei 45°C Badtemperatur und unter 15 mbar Vakuum auf 16,0 g eingeengt, dann ein Gemisch aus 19,75 g Wasser und 2,87 g Aceton hinzugegeben und innerhalb von 60 min auf 5°C abgekühlt. Die entstandene Suspension wurde für 90 min bei 5°C Innentemperatur gerührt, abgenutscht und mit 23 mL kaltem Wasser und 18 mL Ethylacetat gewaschen. Nach Trocknen im Vakuum erhielt man als Hauptprodukt 3,58 g 4-[5-[Bis(2-chlorethyl)amino]-1-methyl-1*H*-benzo[d]imidazol-2-yl]butansäure (= 70% Ausbeute bezogen auf die eingesetzten 5,0 g 4-[5-[Bis(2-hydroxyethyl)amino]-1-methyl-1*H*-benzo[d]imidazol-2-yl]butansäureethylester).

Die beim Abnutschen gewonnene Mutterlauge auf Basis von Aceton/Wasser wurde unter Vakuum vom Aceton befreit und mittels 0,1M NaOH wurde ein pH-Wert zwischen 3 und 4 eingestellt. Die so erhaltene schwach saure Lösung wurde mittels Reversed-Phase Chromatographie (stationäre Phase: DuPont^{™} AmberChrom^{™} CG161M Chromatography Resin) behandelt. Als Elutionsmittel wurde eine 0,1 gew.-%ige wässrige Trifluoressigsäurelösung mit einem von 0 auf 50 Gew.-% ansteigenden Gradienten an Acetonitril verwendet. Das Eluat wurde im Vakuum vom organischen Lösemittel befreit und danach mittels Lyophilisierung getrocknet. Das lyophilisierte Material wurde mit Ethylacetat versetzt, und daraus umkristallisiert.

Man erhielt 0,25 g 4-[5-[Bis(2-chlorethyl)amino]-4-formyl-1-methyl-1*H*-benzo[d]imidazol-2-yl]butansäure (= 4,5 % Ausbeute bezogen auf die eingesetzten 5,0 g 4-[5-[Bis(2-hydroxyethyl)amino]-1-methyl-1H-benzo[d]imidazol-2-yl]butansäureethylester) als Nebenprodukt in Form eines gelben Feststoffs.

Der gelbe Feststoff wurde mittels UV-VIS-, HRMS-, ¹H-NMR- und ¹³C-NMR-Spektroskopie-, als 4-[5-[Bis(2-chlorethyl)amino]-4-formyl-1-methyl-1*H*-benzo[d]imidazol-2-yl]butansäure identifiziert.

UV-VIS in Acetonitril/Wasser mit 0,1% HCOOH (nm): 249 (Peak, breit), 295 (Schulter), 405 (Peak, breit).

HRMS: C₁₇H₂₁O₃N₃Cl₂, 386.10298 (delta m = - 0,76 ppm)

### NMR-Daten (in CDCl₃):

| Position im Ringsystem oder Molekülfragment | ¹H-NMR (ppm, Intensität, Multiplizität) | ¹³C-NMR (ppm) |
|---|---|---|
| 2 | -- | 156.2 |
| 3a | -- | 130.3 |
| 4 | -- | 118.9 |
| 5 | -- | 151.7 |
| 6-CH | 7.51, 1H, dublett | 122.2 |
| 7-CH | 7.93, 1H, dublett | 118.7 |
| 7a | -- | 130.6 |
| NCH₃ | 4.03, 3H, singulett | 31.3 |
| **CH₂**CH₂CH₂COOH | 3.30, 2H, triplett | 24.6 |
| **CH₂**CH₂CH₂COOH | 2.11, 2H, multiplett | 22.8 |
| **CH₂**CH₂CH₂COOH | 2.49, 2H, triplett | 32.8 |
| COOH | breit | 174.1 |
| Cl(CH₂**CH₂**)₂N | 3.70, 4H, triplett | 57.3 |
| Cl(**CH₂**CH₂)₂N | 3.60, 4H, triplett | 41.9 |
| -CHO | 10.7, 1H, singulett | 190.2 |

### Beispiel 3

Ausgewählte Zelllinien wurden 72 Stunden lang bei 37 °C mit verschiedenen Konzentrationen der 4-[5-[Bis(2-chlorethyl)amino]-4-formyl-1-methyl-1*H*-benzo[d]imidazol-2-yl]butansäure (Konzentrationen im Bereich von 1,5 µmol bis 5 mmol pro Liter) inkubiert. Die Zellen wurden dann 1 Stunde lang mit MTS-Lösung (Promega) inkubiert, und die Auswirkungen der 4-[5-[Bis(2-chlorethyl)amino]-4-formyl-1-methyl-1H-benzo[d]imidazol-2-yl]butansäure auf die Zellproliferation wurden kolorimetrisch bei 490 nm bestimmt. Das Zellwachstum wurde in Prozent der entsprechenden Kontrolle (0,5 Gew.-% Dimethylsulfoxid in Wasser) angegeben. Anhand dieser Daten wurden die jeweiligen IC50-Werte berechnet.

| **Organ** | **Zelllinie** | **IC50 (mol pro Liter)** |
|---|---|---|
| Haut | A375 | 1,3 · 10⁻³ |
| Nieren | Caki-1 | 1,6 · 10⁻³ |
| Lunge | Calu-6 | 1,3 · 10⁻³ |
| Gehirn | U87MG | 2,9 · 10⁻³ |
| Bauchspeicheldrüse | BxPC-3 | 7,4 · 10⁻⁴ |

## Patentansprüche

1. Verfahren zur Herstellung eines 4-[5-[Bis(2-chlorethyl)amino]-1-methyl-1*H-*benzo[d]imidazol-2-yl]butansäurealkylesters, wobei ein 4-[5-[Bis(2-hydroxyethyl)amino]-1-methyl-1*H*-benzo[d]imidazol-2-yl]butansäurealkylester mit Chlormethylendimethyliminiumchlorid umgesetzt wird.

2. Verfahren nach Anspruch 1, wobei der 4-[5-[Bis(2-hydroxyethyl)amino]-1-methyl-1*H-*benzo[d]imidazol-2-yl]butansäurealkylester unter C₁₋₄-Alkylestern ausgewählt ist.

3. Verfahren nach Anspruch 1 oder 2, wobei der 4-[5-[Bis(2-hydroxyethyl)amino]-1-methyl-1*H*-benzo[d]imidazol-2-yl]butansäureethylester verwendet wird.

4. Verfahren nach einem der vorhergehenden Ansprüche, wobei mit einem Molverhältnis im Bereich von 2,0 bis 2,2 mol Chlormethylendimethyliminiumchlorid pro mol 4-[5-[Bis(2-hydroxyethyl)amino]-1-methyl-1*H*-benzo[d]imidazol-2-yl]butansäurealkylester gearbeitet wird.

5. Verfahren nach Anspruch 1, wobei mit einem Molverhältnis im Bereich von >2,2 bis 5 mol Chlormethylendimethyliminiumchlorid pro mol 4-[5-[Bis(2-hydroxyethyl)amino]-1-methyl-1*H-*benzo[d]imidazol-2-yl]butansäurealkylester gearbeitet wird.

6. Als Nebenprodukt des Verfahrens nach Anspruch 5 erhältlicher 4-[5-[Bis(2-chlorethyl)amino]-4-formyl-1-methyl-1*H*-benzo[d]imidazol-2-yl]butansäurealkylester.

7. Verfahren nach Anspruch 2, wobei mit einem Molverhältnis im Bereich von >2,2 bis 5 mol Chlormethylendimethyliminiumchlorid pro mol 4-[5-[Bis(2-hydroxyethyl)amino]-1-methyl-1*H-*benzo[d]imidazol-2-yl]butansäure-C₁₋₄-alkylester gearbeitet wird.

8. Als Nebenprodukt des Verfahrens nach Anspruch 7 erhältlicher 4-[5-[Bis(2-chlorethyl)amino]-4-formyl-1-methyl-1*H*-benzo[d]imidazol-2-yl]butansäure-C₁₋₄-alkylester.

9. Verfahren nach Anspruch 3, wobei mit einem Molverhältnis im Bereich von >2,2 bis 5 mol Chlormethylendimethyliminiumchlorid pro mol 4-[5-[Bis(2-hydroxyethyl)amino]-1-methyl-1*H-*benzo[d]imidazol-2-yl]butansäureethylester gearbeitet wird.

10. Als Nebenprodukt des Verfahrens nach Anspruch 9 erhältlicher 4-[5-[Bis(2-chlorethyl)amino]-4-formyl-1-methyl-1*H*-benzo[d]imidazol-2-yl]butansäureethylester.

11. Verfahren nach einem der Ansprüche 5, 7 oder 9, wobei man das dabei erhaltene Verfahrensnebenprodukt einer anschließenden Esterhydrolyse unterwirft unter Bildung von 4-[5-[Bis(2-chlorethyl)amino]-4-formyl-1-methyl-1*H*-benzo[d]imidazol-2-yl]butansäure.

12. Verfahren nach Anspruch 11, wobei das Verfahrensnebenprodukt der Esterhydrolyse gemeinsam mit dem Verfahrenshauptprodukt unterworfen wird.

13. Als Produkt des Verfahrens nach Anspruch 11 oder 12 erhältliche 4-[5-[Bis(2-chlorethyl)amino]-4-formyl-1-methyl-1*H*-benzo[d]imidazol-2-yl]butansäure.

14. 4-[5-[Bis(2-chlorethyl)amino]-4-formyl-1-methyl-1*H*-benzo[d]imidazol-2-yl]butansäure oder ein pharmazeutisch verträgliches Salz davon zur Verwendung in der Medizin.

## Claims

1. A method for preparing a 4-[5-[bis(2-chloroethyl)amino]-1-methyl-1*H*-benzo[d]imidazol-2-yl]butyric acid alkyl ester, wherein a 4-[5-[bis(2-hydroxyethyl)amino]-1-methyl-1*H-*benzo[d]imidazol-2-yl]butyric acid alkyl ester is reacted with chloromethylenedimethyliminium chloride.

2. The method according to claim 1, wherein the 4-[5-[bis(2-hydroxyethyl)amino]-1-methyl-1*H*-benzo[d]imidazol-2-yl]butyric acid alkyl ester is selected from C₁₋₄alkyl esters.

3. The method according to claim 1 or 2, wherein the 4-[5-[bis(2-hydroxyethyl)amino]-1-methyl-1*H*-benzo[d]imidazol-2-yl]butyric acid ethyl ester is used.

4. The method according to any of the preceding claims, wherein a molar ratio in the range from 2.0 to 2.2 mol of chloromethylenedimethyliminium chloride per mol of 4-[5-[bis(2-hydroxyethyl)amino]-1-methyl-1*H*-benzo[d]imidazol-2-yl]butyric acid alkyl ester is used.

5. The method according to claim 1, wherein a molar ratio in the range from >2.2 to 5 mol of chloromethylenedimethyliminium chloride per mol of 4-[5-[bis(2-hydroxyethyl)amino]-1-methyl-1*H*-benzo[d]imidazol-2-yl]butyric acid alkyl ester is used.

6. A 4-[5-[bis(2-chloroethyl)amino]-4-formyl-1-methyl-1*H*-benzo[d]imidazol-2-yl]butyric acid alkyl ester obtainable as a by-product of the method according to claim 5.

7. The method according to claim 2, wherein a molar ratio in the range from >2.2 to 5 mol of chloromethylenedimethyliminium chloride per mol of 4-[5-[bis(2-hydroxyethyl)amino]-1-methyl-1*H*-benzo[d]imidazol-2-yl]butyric acid C₁₋₄ alkyl ester is used.

8. A 4-[5-[bis(2-chloroethyl)amino]-4-formyl-1-methyl-1*H*-benzo[d]imidazol-2-yl]butyric acid C₁₋₄ alkyl ester obtainable as a by-product of the method according to claim 7.

9. The method according to claim 3, wherein a molar ratio in the range from >2.2 to 5 mol of chloromethylenedimethyliminium chloride per mol of 4-[5-[bis(2-hydroxyethyl)amino]-1-methyl-1*H*-benzo[d]imidazol-2-yl]butyric acid ethyl ester is used.

10. A 4-[5-[bis(2-chloroethyl)amino]-4-formyl-1-methyl-1*H*-benzo[d]imidazol-2-yl]butyric acid ethyl ester obtainable as a by-product of the method according to claim 9.

11. The method according to any of claims 5, 7 or 9, wherein the method by-product obtained thereby is subjected to a subsequent ester hydrolysis to form 4-[5-[bis(2-chloroethyl)amino]-4-formyl-1-methyl-1*H*-benzo[d]imidazol-2-yl]butyric acid.

12. The method according to claim 11, wherein the method by-product is subjected to ester hydrolysis together with the main method product.

13. A 4-[5-[bis(2-chloroethyl)amino]-4-formyl-1-methyl-1*H*-benzo[d]imidazol-2-yl]butyric acid obtainable as the product of the method according to claim 11 or 12.

14. A 4-[5-[bis(2-chloroethyl)amino]-4-formyl-1-methyl-1*H*-benzo[d]imidazol-2-yl]butyric acid or a pharmaceutically acceptable salt thereof for use in medicine.

## Revendications

1. Procédé pour la préparation d'un ester alkylique d'acide 4-[5-[bis(2-chloroéthyl)amino]-1-méthyl-1*H*-benzo[d]imidazol-2-yl]butanoïque, dans lequel un ester alkylique d'acide 4-[5-[bis(2-hydroxyéthyl)amino]-1-méthyl-1*H*-benzo[d]imidazol-2-yl]butanoïque est mis à réagir avec du chlorure de chlorométhylènediméthyliminium.

2. Procédé selon la revendication 1, dans lequel l'ester alkylique d'acide 4-[5-[bis(2-hydroxyéthyl)amino]-1-méthyl-1*H*-benzo[d]imidazol-2-yl]butanoïque est choisi parmi des esters alkyliques en C₁₋₄.

3. Procédé selon la revendication 1 ou 2, dans lequel l'ester éthylique d'acide 4-[5-[bis(2-hydroxyéthyl)amino]-1-méthyl-1*H*-benzo[d]imidazol-2-yl]butanoïque est utilisé.

4. Procédé selon l'une des revendications précédentes, dans lequel on travaille avec un rapport molaire dans la plage allant de 2,0 à 2,2 moles de chlorure de chlorométhylènediméthyliminium par mole d'ester alkylique d'acide 4-[5-[bis(2-hydroxyéthyl)amino]-1-méthyl-1*H*-benzo[d]imidazol-2-yl]butanoïque.

5. Procédé selon la revendication 1, dans lequel on travaille avec un rapport molaire dans la plage allant de > 2,2 à 5 moles de chlorure de chlorométhylènediméthyliminium par mole d'ester alkylique d'acide 4-[5-[bis(2-hydroxyéthyl)amino]-1-méthyl-1*H*-benzo[d]imidazol-2-yl]butanoïque.

6. Ester alkylique d'acide 4-[5-[bis(2-chloroéthyl)amino]-4-formyl-1-méthyl-1*H-*benzo[d]imidazol-2-yl]butanoïque pouvant être obtenu comme sous-produit du procédé selon la revendication 5.

7. Procédé selon la revendication 2, dans lequel on travaille avec un rapport molaire dans la plage allant de > 2,2 à 5 moles de chlorure de chlorométhylènediméthyliminium par mole d'ester alkylique en C₁₋₄ d'acide 4-[5-[bis(2-hydroxyéthyl)amino]-1-méthyl-1*H-*benzo[d]imidazol-2-yl]butanoïque.

8. Ester alkylique en C₁₋₄ d'acide 4-[5-[bis(2-chloroéthyl)amino]-4-formyl-1-méthyl-1*H-*benzo[d]imidazol-2-yl]butanoïque pouvant être obtenu comme sous-produit du procédé selon la revendication 7.

9. Procédé selon la revendication 3, dans lequel on travaille avec un rapport molaire dans la plage allant de > 2,2 à 5 moles de chlorure de chlorométhylènediméthyliminium par mole d'ester éthylique d'acide 4-[5-[bis(2-hydroxyéthyl)amino]-1-méthyl-1*H*-benzo[d]imidazol-2-yl]butanoïque.

10. Ester éthylique d'acide 4-[5-[bis(2-chloroéthyl)amino]-4-formyl-1-méthyl-1*H-*benzo[d]imidazol-2-yl]butanoïque pouvant être obtenu comme sous-produit du procédé selon la revendication 9.

11. Procédé selon l'une des revendications 5, 7 ou 9, dans lequel on soumet le sous-produit de procédé ainsi obtenu à une hydrolyse d'ester subséquente avec formation d'acide 4-[5-[bis(2-chloroéthyl)amino]-4-formyl-1-méthyl-1*H*-benzo[d]imidazol-2-yl]butanoïque.

12. Procédé selon la revendication 11, dans lequel le sous-produit de procédé est soumis à l'hydrolyse d'ester conjointement avec le produit principal de procédé.

13. Acide 4-[5-[bis(2-chloroéthyl)amino]-4-formyl-1-méthyl-1*H*-benzo[d]imidazol-2-yl]butanoïque pouvant être obtenu comme produit du procédé selon la revendication 11 ou 12.

14. Acide 4-[5-[bis(2-chloroéthyl)amino]-4-formyl-1-méthyl-1*H*-benzo[d]imidazol-2-yl]butanoïque ou sel pharmaceutiquement acceptable de celui-ci, destiné à être utilisé dans le domaine de la médecine.
